# EUROPEAN PATENT APPLICATION

(11) **EP 1 777 209 A1**
(43) Date of publication of application: **25.04.2007**
(21) Application number: 05022865.9
(22) Date of filing: 20.10.2005
(51) Int. Cl.: C07C 2/08, C07C 7/00, B01J 8/00

(54) **Method for oligomerization of ethylene and reactor system therefor with removal of heavy molecular weight oligomers**

(71) Applicant: Saudi Basic Industries Corporation, Riyadh 11422 (SA); Linde AG, 65189 Wiesbaden (DE)
(72) Inventor: Schneider, Richard, 82449 Uffing (DE); Fritz, Peter M., 82008 Unterhaching (DE); Muschelknantz, Sebastian, 81479 München (DE); Bölt, Heinz, 82515 Wolfratshausen (DE); Ali, Talal, 11422 Riyadh (SA); Mousa, Fuad, 11422 Riyadh (SA)
(74) Representative: Winkler, Andreas Fritz Ernst

(57) **Abstract**

The present invention relates to a method for oligomerization of ethylene to form linear alpha-olefins in an oligomerization reactor in the presence of solvent and catalyst, wherein a reaction product containing heavy molecular weight oligomers (HMWO) is discharged from the oligomerization reactor and is passed to and filtrated in a filtration unit, characterized in that the filtration residue of HMWO is removed from the filtration unit discontinuously via a discharge valve; and to a reactor system therefore.

## Description

The present invention relates to a method for oligomerization of ethylene to form linear alpha-olefins in an oligomerization reactor in the presence of solvent and catalyst, wherein a reaction product containing heavy molecular weight oligomers (HMWO) is discharged from the oligomerization reactor and is passed to and filtrated in a filtration unit, and to a reactor system therefore.

Methods for oligomerization of ethylene are widely known in the art. For example, DE 43 38 414 C1 discloses a process for the preparation of linear alpha-olefins by oligomerization of ethylene, wherein oligomerization takes place in the presence of an organic solvent and a homogenous liquid catalyst. Usually, a catalyst is utilized in that process comprising a zirconium component and an organoaluminum component which acts as a cocatalyst. From the oligomerization reactor gaseous and liquid reaction products are discharged and further processed. The liquid reaction product may, amongst solvent, catalyst, dissolved ethylene and liquid linear alpha-olefins, also comprise heavy linear alpha-olefins having more than 20 carbon atoms which are more or less solid. These undesired by-product have to be removed from the liquid reaction product prior to further processing thereof. Usually, the solid heavy molecular weight oligomers are separated from the liquid reaction product by filtration in a filtration unit. The filtration residue of HMWO has to be removed from the filtration unit, i.e. a specific amount of HMWO shall be removed per unit of time. According to the prior art, this amount has been so far removed continuously via a small valve having a very small opening and employing a high differential pressure.

Such small valve openings have, however, a high tendency for plugging and result in significant maintenance efforts and reduced availability of the reactor system.

It is an object of the present invention to provide a method for oligomerisation of ethylene which overcomes the drawbacks of the prior art. Especially plugging of the discharge valve of the filtration unit for separating the heavy molecular weight oligomers from the liquid reaction product shall be avoided to reduce maintenance efforts and increase reactor availability. Additionally, it is an object to provide a reactor system which may be utilized in the inventive method.

The first object is achieved in that the filtration residue of HMWO is removed from the filtration unit discontinuously via a discharge valve.

Preferably, opening and closing of the discharge valve is controlled by a timer, preferably periodically.

In one embodiment the discharge valve has a diameter of about 1 to 2 inch, preferably 1 inch, each with full-size internals.

Preferably, a restriction orifice is installed downstreams of the discharge valve.

The differential pressure between both sides of the discharge valve is adjusted at a certain level at about 1 to 20 bar, preferably 1 to 5 bar, more preferably 1 to 2 bar.

Preferably, heavy molecular weight oligomers (HMWO) are oligomers of ethylene with a carbon number of C₂₀₊.

The object is also achieved by a reactor system for oligomerization of ethylene to form linear alpha-olefins, preferably utilizing an inventive method, comprising a reactor and a filtration unit to remove heavy molecular weight oligomers from reaction product, the filtration unit comprising a discharge valve which is openable and closeable discontinuously.

Surprisingly, it was found that utilizing the inventive method a specific amount of heavy molecular weight oligomers per unit of time may be emptied from the filtration unit which is identical to the amount being so far removed continuously by prior art methods. However, in contrast to the prior art, the filtration residue of HMWO is now removed discontinuously via a much larger discharge valve with a larger opening, the opening and closing thereof is preferably controlled by a timer, more preferably periodically. The filtration residue is removed discontinuously, the differential pressure between both sides of the discharge valve may be reduced and controlled at a certain level, also allowing enlargement of the valve diameter and / or valve opening. Thus, plugging of the discharge valve may be successfully excluded, maintenance efforts may be reduced and reactor availability may be increased.

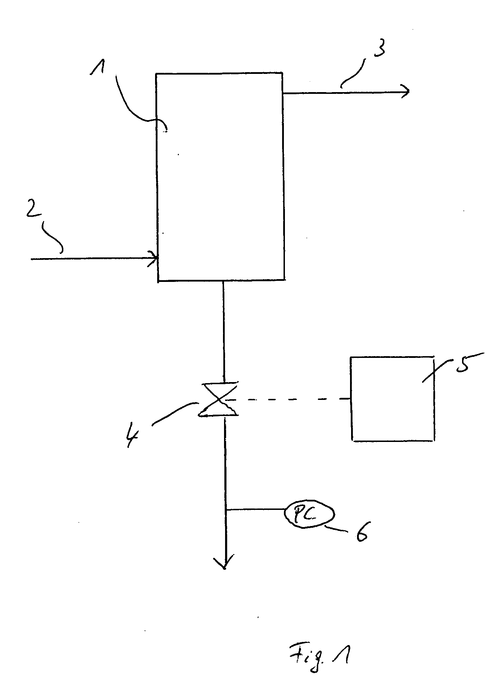

Additional features and advantages of the inventive method are further illustrated with reference to the accompanying drawing, wherein figure 1 illustrates a filtration unit which may be utilized in the inventive method.

Figure 1 illustrates a filtration unit 1 which may be utilized in a reactor system for the oligomerization of ethylene. From an oligomerization reactor (not shown) a liquid reaction product comprising solvent, dissolved monomers, catalyst, cocatalyst and liquid linear alpha-olefins as well as a solid heavy molecular weight olefins (HMWO) is transferred to the filtration unit 1 via line 2. Via line 3 the liquid components (solvent, dissolved monomer, catalyst, cocatalyst and liquid linear alpha-olefins) are removed and can be passed to a further processing unit (not shown). From the filtration unit 1 the filtration residue of HMWO has to be removed. Especially, a specific and constant amount per unit of time shall be removed. Removal of the filtration residue is accomplished in a discontinuous manner by opening and closing a discharge valve 4 opening and closing thereof is controlled by a timer 5. The timer 5 may be in communication with a pressure control device 6. The discharge valve 4 is opened discontinuously, the differential pressure between both sides of the discharge valve 4 may be significantly reduced and controlled, also allowing an enlarged valve diameter and / or valve opening. Since the valve diameter and / or valve opening is enlarged, plugging thereof can be successfully excluded.

The features disclosed in the foregoing description, in the claims and in the drawing may, both separately and in any combination thereof, be material for realizing the invention in diverse forms thereof.

## Claims

1. Method for oligomerization of ethylene to form linear alpha-olefins in an oligomerization reactor in the presence of solvent and catalyst, wherein a reaction product containing heavy molecular weight oligomers (HMWO) is discharged from the oligomerization reactor and is passed to and filtrated in a filtration unit (1), **characterized in that** the filtration residue of HMWO is removed from the filtration unit (1) discontinuously via a discharge valve (4).

2. Method according to claim 1, wherein opening and closing of the discharge valve (4) is controlled by a timer (5), preferably periodically.

3. Method according to claim 1 or 2, wherein the discharge valve (4) has a diameter of about 1 to 2 inch, preferably 1 inch, each with full-size internals.

4. Method according to any of the preceding claims, wherein a restriction orifice is installed downstreams of the discharge valve (4).

5. Method according to any of the preceding claims, wherein the differential pressure between both sides of the discharge valve (4) is controlled at about 1 to 20 bar, preferably 1 to 5 bar, more preferably 1 to 2 bar.

6. Method according to any of the preceding claims, wherein heavy molecular weight oligomers (HMWO) are oligomers of ethylene with a carbon number of C₂₀+

7. Reactor system for oligomerization of ethylene to form linear alpha-olefins, preferably utilizing a method according to any of the preceding claims 1 to 6, comprising a reactor and a filtration unit (1) to remove heavy molecular weight oligomers from reaction product, the filtration unit (1) comprising a discharge valve (4) which is openable and closeable discontinuously.
